# EUROPEAN PATENT APPLICATION

(11) **EP 1 421 903 A1**
(43) Date of publication of application: **26.05.2004**
(21) Application number: 03025121.9
(22) Date of filing: 03.11.2003
(51) Int. Cl.: A61B 5/087, G01F 1/44

(54) **Arrangement for passive gas sampling**

(30) Priority: 20.11.2002 SE 0203428
(71) Applicant: Maquet Critical Care AB, 171 95 Solna (SE)
(72) Inventor: Castor, Rolf, 129 42 Hägersten (SE); Hallbäck, Magnus, 167 38 Bromma (SE)
(74) Representative: Samzelius, Roger Mikael

(57) **Abstract**

An arrangement (2) for passive gas sampling in a breathing system is described. The arrangement (2; 14) comprises a conduit (4) having a venturi-nozzle (8) for passively generating pressure variations in the conduit (4) dependent on a flow of breathing gas in the conduit (4) and a first port (10) and a second port (12) terminating in the conduit (4) and connected to a measurement chamber (6), wherein the first port (10) and the second port (12) are arranged relative to the venturi-nozzle (8) such that the pressure variations force a flow of breathing gas through the first port (10) into the measurement chamber (6) and out through the second port (12).

## Description

The present invention relates to an arrangement for passive gas sampling according to the preamble of claim 1.

In breathing systems such as ventilators and anaesthetic apparatus the breathing gas is analysed regularly. This may be done directly in the main supply (with a so-called mainstream-analyser) or by diverting a gas sample to a measuring chamber (so-called sidestream-analyser).

The diversion of the gas sample can be done actively by means of a pump or the like or passively, for example by creating a pressure variation between the pressure chamber's inlet and outlet. An example of the latter can be found described in US 6,450,968.

An aim of the present invention is to provide an alternative embodiment of an arrangement for passive gas sampling.

This aim is achieved according to the invention by means of the arrangement according to above being adapted as evidenced by the characterising portion of claim 1.

Pressure differences along a relatively short length can be generated with a venturi-nozzle in a conduit. This pressure difference can be employed to passively lead a gas sample through a first port to a measurement chamber. Simultaneously, an earlier gas sample can be lead via a second port from the measurement chamber back to the conduit.

Exemplary embodiments of arrangements according to the present invention shall now be described with reference to the accompanying figures, of which:
FIG. 1 shows a first embodiment of an arrangement according to the invention; and
FIG.2 shows a second embodiment of an arrangement according to the invention.

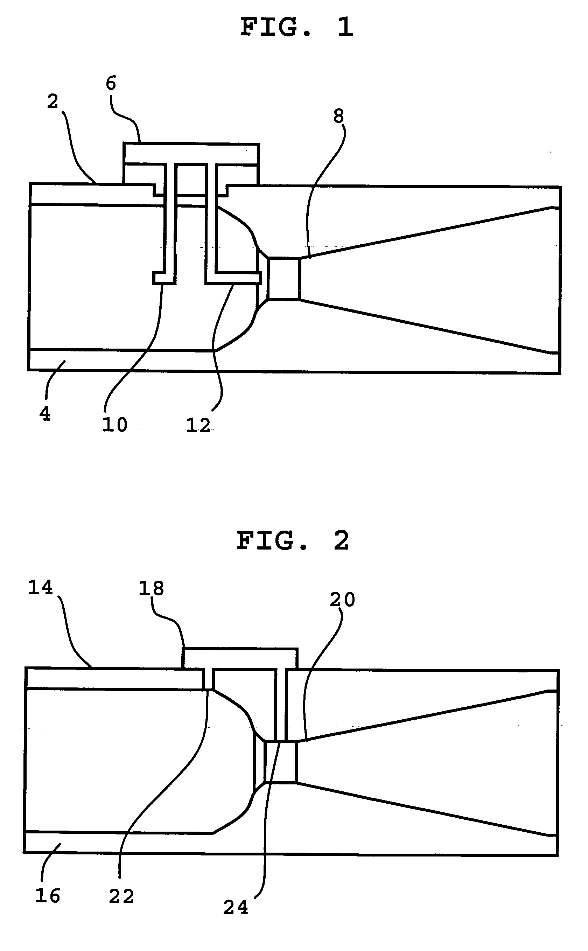

A first embodiment of an arrangement 2 according to the invention is shown in FIG. 1. The arrangement 2 comprises a conduit 4 through which a breathing gas can flow (it is irrelevant to the invention whether it is an inspiration flow or an expiration flow). A gas sample can be passively transferred from the breathing gas flow to a measurement chamber 6 for analysis. The analysis can be achieved in any known manner, for example optically, acoustically or electrochemically.

In order to achieve a passive sampling-flow to the measurement chamber 6 a venturi-nozzle 8 is arranged in the conduit 4. A first port 10 and a second port 12, both connected to the measurement chamber 6, are arranged in the conduit 4 so that the pressure variation produced by the venturi-nozzle 8 can be optimally utilized to obtain the sampling-flow into the measurement chamber 6 through the first port 10. An earlier sample will, at the same time, be conducted out of the measurement chamber 6 through the second port 12 and back to the breathing gas flow in the conduit 4.

The gas sample in the measurement chamber 6 will, in principle, be exchanged once every breathing cycle, during inspiration or expiration. The analysis in the measurement chamber 6 may be done continuously when a new gas sample flows into the measurement chamber 6 or during the pause that is created when a change of sample does not occur (expiration or inspiration).

A variation is shown in FIG. 2 where an arrangement 14 according to the invention comprises a conduit 16, a measurement chamber 18, a venturi-nozzle 20, a first port 22 and a second port 24.

The difference is, in principle, that in the arrangement 14 according to the second exemplary embodiment the ports 22,24 comprise, in principle, holes in the tube 16 having openings strategically located to utilize the pressure variation generated by the venturi-nozzle.

## Claims

1. An arrangement (2; 14) for passive gas sampling in a breathing system **characterised in that** the arrangement (2; 14) comprises a conduit (4; 16) having a venturi-nozzle (8; 20) for passively generating pressure variations in the conduit (4; 16) dependent on a flow of breathing gas in the conduit (4; 16) and a first port (10; 22) and a second port (12; 24) terminating in the conduit (4; 16) and connected to a measurement chamber (6; 18), wherein the first port (10; 22) and the second port (12; 24) are arranged relative to the venturi-nozzle (8;20) such that the pressure variations forces a flow of breathing gas through the first port (10;22) into the measurement chamber (6;18) and out through the second port (12;24).
